(19) Europäisches Patentamt
European Patent Office
Office européen des brevets

(11) Veröffentlichungsnummer: **0 276 393 B1**

# (12) EUROPÄISCHE PATENTSCHRIFT

(45) Veröffentlichungstag der Patentschrift: **04.03.92**

(51) Int. Cl.$^5$: **C07J 5/00**, C07J 7/00, C07J 71/00

(21) Anmeldenummer: **87116855.5**

(22) Anmeldetag: **14.11.87**

## (54) Verfahren zur Herstellung von 6Alpha, 9Alpha-Difluor-11Beta, 17Alpha-dihydroxy-16Alpha-methyl-4-pregnen-3,20-dion und dessen Derivate.

(30) Priorität: **28.11.86 DE 3640709**

(43) Veröffentlichungstag der Anmeldung: **03.08.88 Patentblatt 88/31**

(45) Bekanntmachung des Hinweises auf die Patenterteilung: **04.03.92 Patentblatt 92/10**

(84) Benannte Vertragsstaaten: **AT BE CH DE ES FR GB GR IT LI LU NL SE**

(56) Entgegenhaltungen:
**GB-A- 903 049**
**GB-A- 924 931**
**US-A- 2 658 023**
**US-A- 3 499 016**

**JOURNAL OF THE AMERICAN CHEMICAL SOCIETY, Band 86, Nr. 19, 5. Oktober 1964, Seiten 4011-4016, Washington, DC, US, A.D. CROSS:"Steroids, CCLXII. Spectra and stereochemistry. XVI. A study of the mechanism of longrange 19-proton-6beta-fluroine coupling in 6beta-fluorosteroids"**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Hempel, Gerhard, Dr.**
**Jahnstrasse 34**
**W-4712 Werne(DE)**
Erfinder: **Kennecke, Mario, Dr.**
**Taubertstrasse 31f**
**W-1000 Berlin 33(DE)**
Erfinder: **Krieger, Bernhard, Dr.**
**Martinstrasse 28**
**W-4750 Unna(DE)**
Erfinder: **Philippson, Rainer, Dr.**
**Hochstrasse 63**
**W-4709 Bergkamen-Mitte(DE)**
Erfinder: **Triem, Hermann, Dr.**
**Zeisigweg 10**
**W-4700 Hamm(DE)**
Erfinder: **Weber, Alfred, Dr.**
**Schützallee 56**
**W-1000 Berlin 37(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**JOURNAL OF THE AMERICAN CHEMICAL SOCIETY, Band 82, Nr. 9, 5. Mai 1960, Seiten 2318-2322, Washington, DC, US; J.A. EDWARDS et al.: "Steroids. CXXXVI: Synthesis of a new class of potent cortical hormones. 6alpha-fluoro- and 6alpha-9alpha-difluoro-16alpha-methylprednisolone and related steroids"**

**Beschreibung**

Die Erfindung betrifft ein Verfahren zur Herstellung von 6α,9α-Difluor-11β,17α-dihydroxy-4-pregnen-3,20-dion und dessen Derivaten der allgemeinen Formel I

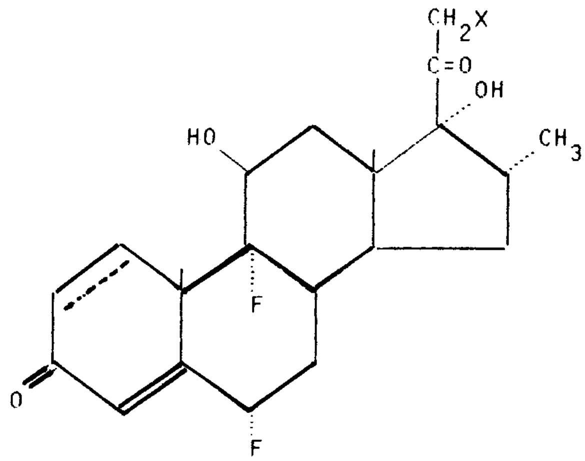

(I),

worin

..... eine Einfachbindung oder eine Doppelbindung symbolisiert und

X ein Wasserstoffatom, ein Bromatom, ein Jodatom oder eine Alkanoyloxygruppe mit maximal 8 Kohlenstoffatomen darstellt, welches dadurch gekennzeichnet ist, daß man das 3β,17α-Dihydroxy-16α-methyl-5-pregnen20-on der Formel II

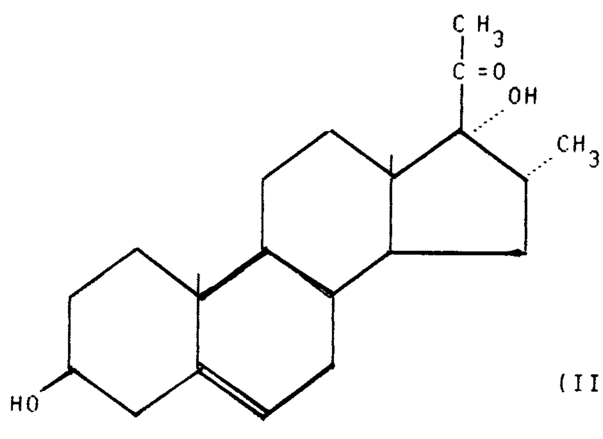

(II),

in Gegenwart von Harnstoff mit N-Brom-succinimid und Fluorwasserstoff umsetzt, das entstandene 5α-Brom-6β-fluor-3β,17α-dihydroxy-16α-methyl-pregnan-20-on der Formel III

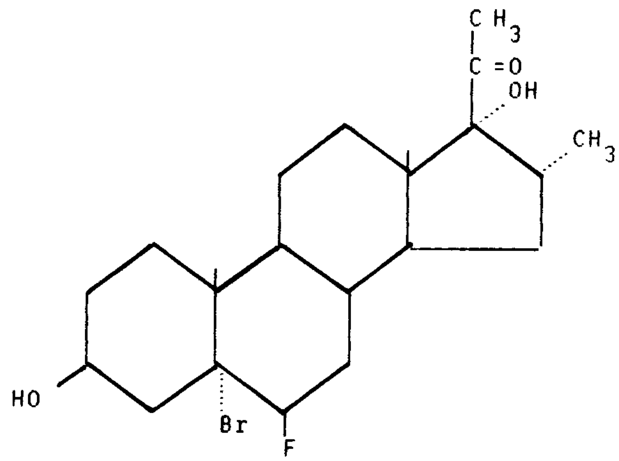

(III),

mittels Chromsäure zum 5α-Brom-6β-fluor-17α-hydroxy-16α-methyl-pregnan-3,20-dion der Formel IV

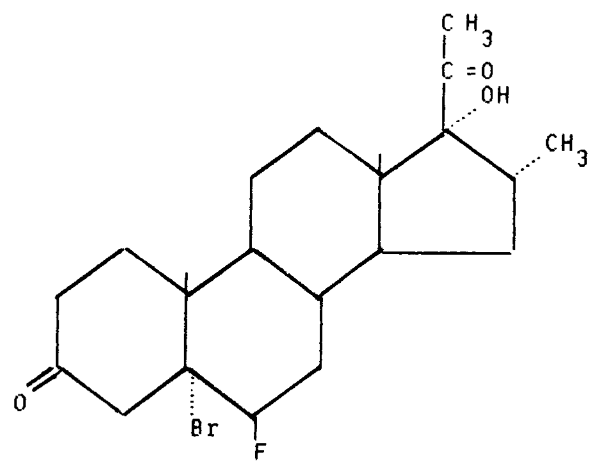

(IV),

oxidiert und aus diesem durch Behandeln mit einer starken Säure in einem polarem Lösungsmittel Bromwasserstoff abspaltet, wobei gleichzeitig eine Isomerisierung am C-6 stattfindet, das gebildete 6α-Fluor-17α-hydroxy-16α-methyl-4-pregnen-3,20-dion der Formel V

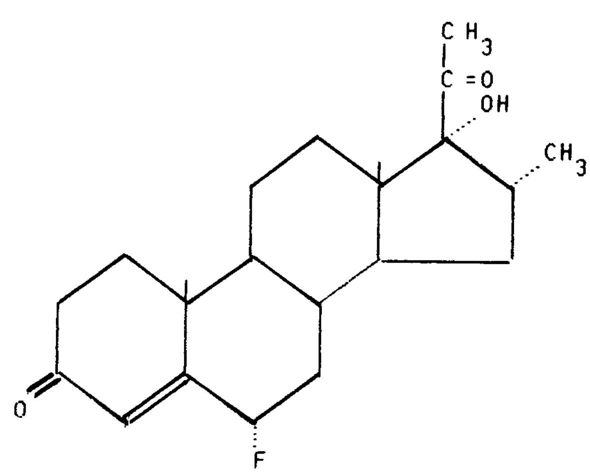

(V),

mit einer lebenden Kultur von Curvularia lunata in der 11-Position zum 6α-Fluor-11β,17α-dihydroxy-16α-methyl-4-pregnen-3,20-dion der Formel VI

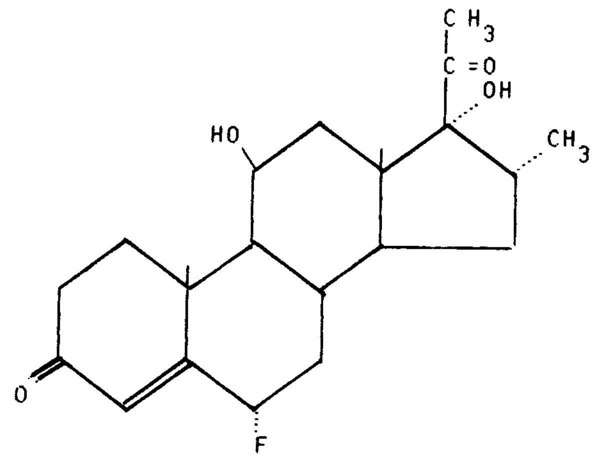

(VI),

hydroxyliert und aus diesem Wasser abspaltet, an das entstandene 6α-Fluor-17α-hydroxy-16α-methyl-4,(9)-,11-pregnadien-3,20-dion der Formel VII

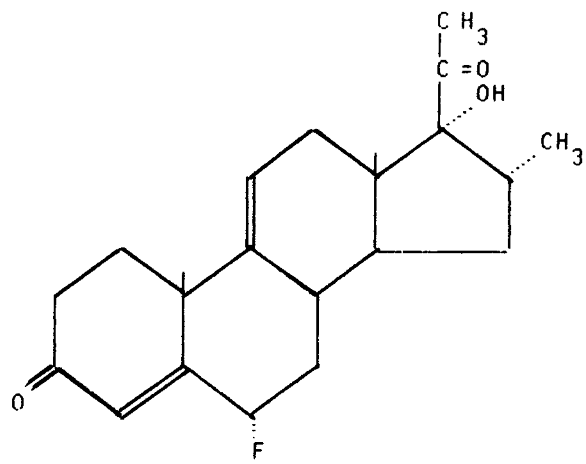

(VII),

BrOH anlagert, aus dem entstandenen 9α-Brom-6α-fluor-11β,17α-dihydroxy-16α-methyl-4-pregnen-3,20-dion der Formel VIII

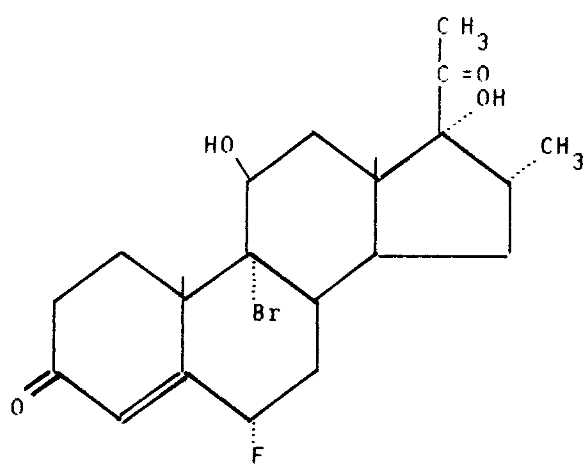

(VIII),

Bromwasserstoff abspaltet und den Epoxidring des gebildeten 9,11β-Epoxy-6α-fluor-17α-hydroxy-16α-methyl-4-pregnen-3,20-dions der Formel IX

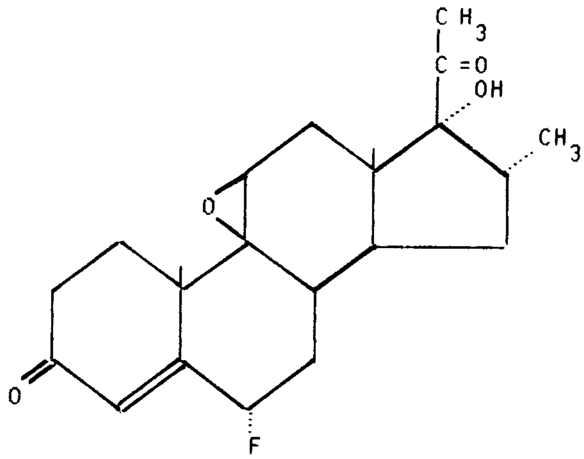

(IX),

mit Fluorwasserstoff öffnet, sowie gewünschtenfalls das erhaltene 6α,9α-Difluor-11β,17α-dihydroxy-16α-methyl-4-pregnen-3,20-dion der Formel Ia

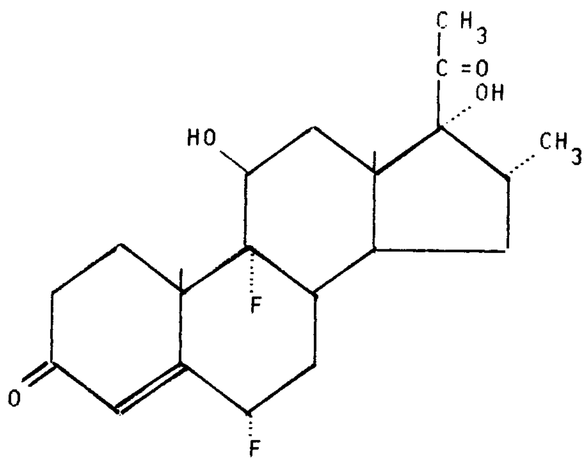

(Ia),

durch Fermentation mittels zur Steroid-$\Delta^1$-dehydrierung befähigten Mikroorganismen zum 6α,9α-Difluor-11β,17α-dihydroxy-16α-methyl-1,4-pregnadien-3,20-dion der Formel Ib

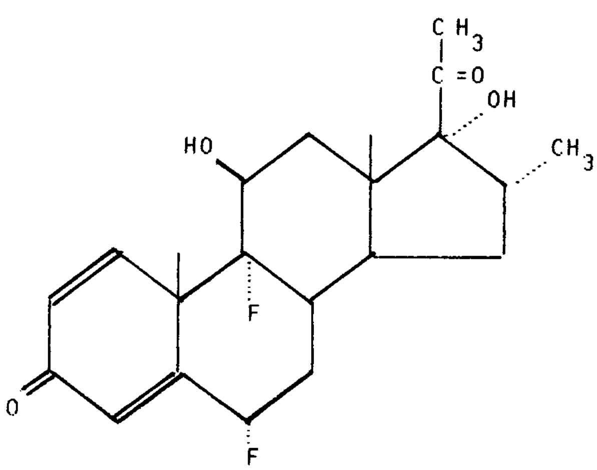

(Ib),

dehydriert, dieses mittels Brom oder Jod in das 6α,9α-Difluor-11β, 17α-dihydroxy-21-halogen-16α-methyl-1,4-pregnadien-3,20-dion der Formel Ic

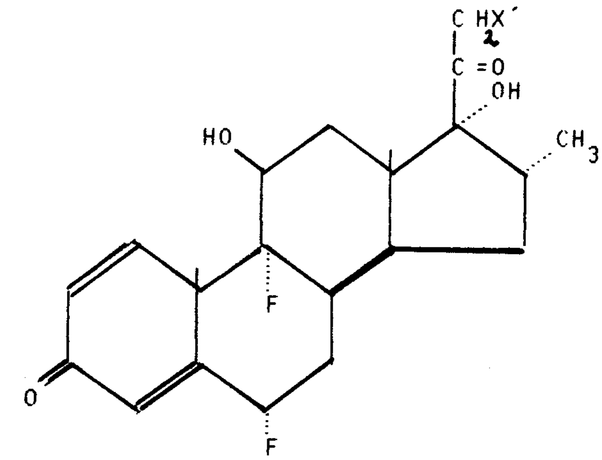

(Ic),

worin X' ein Bromatom oder Jodatom darstellt, überführt und aus diesem durch Austausch des Jods gegen eine Alkanoyloxygruppe das 21-Alkanoyloxy-6α,9α-difluor-11β, 17α-dihydroxy-16α-methyl-1,4-pregnadien-3,20-dion der Formel Id

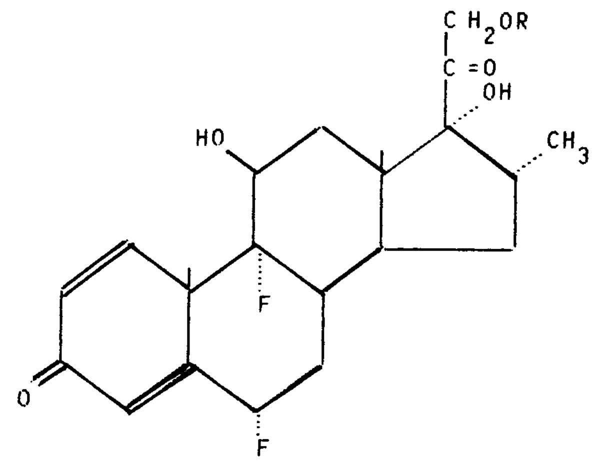

(Id),

worin R eine eine Alkanoylgruppe mit maximal 8 Kohlenstoffatomen bedeutet, darstellt.

Bekanntlich ist das 6α,9α-Difluor-11β,17α,21-trihydroxy-16α-methyl-1,4-pregnadien-3,20-dion (Flumetason) ein Kortikoid-Wirkstoff dessen Synthese sehr aufwendig ist. (J.A. Edwards et. al., J. Amer. Chem. Soc., 82, 1960, 23182322.) Das erfindungsgemäße Verfahren ermöglicht es, dieser Substanz in wesentlich einfacherer Weise und unter Erzielung signifikant höherer Ausbeuten herzustellen, als dies mittels der vorbekannten Synthesen möglich ist.

In der ersten Stufe des erfindungsgemäßen Verfahrens wird 3β,17α-Dihydroxy-16α-methyl-5-pregnen-20-on mit einem N-Bromacylamid (beispielsweise N-Bromacetamid oder insbesondere N-Bromsuccinimid) und Fluorwasserstoff umgesetzt. Führt man diese Reaktion unter den Bedingungen durch, welche man üblicherweise zur Anlagerung eines Brom- und Fluoratoms an die 5(6)-Doppelbindung eines Steroides verwendet (J.Fried et. al. Organic Reactions in Steroid Chemistry, van Nostrand Reinhold, Comp. New York et.al. Vol 1, 1972, 454ff) so erhält man nur geringe Ausbeuten an Verfahrensprodukt. Uberraschend hohe Ausbeuten an erwünschtem 5α-Brom-6β-fluor-3β,17α-dihydroxy-16α-methyl-pregnan-20-on erzielt man hingegen, wenn man die Reaktion in Gegenwart von Harnstoff durchfuhrt. Günstige Bedingungen zur Durchführung dieses Reaktionsschrittes sind beispielsweise folgende:

Das 3β,17α-Dihydroxy-16α-methyl-5-pregnen-20-on wird in einem inerten Lösungsmittel suspendiert, mit einem vorgefertigten Harnstoff-Fluorwasserstoff-Reagenz und anschließend portionsweise mit dem N-Bromacylamid versetzt, wobei die Reaktionstemperatur -20° C bis +10° C beträgt. Geeignete inerte Lösungsmittel sind beispielsweise aromatische Kohlenwasserstoffe, wie Benzol, Toluol, Xylol etc., oder

chlorierte Kohlenwasserstoffe wie Dichlormethan, Trichlormethan oder Tetrachlorethan, geeignete Harnstoff-Fluorwasserstoff-Reagenzien sind solche, die 30 bis 70 Gewichtsprozent Fluorwasserstoff enthalten.

Das so hergestellte 5α-Brom-6β-fluor-3β,17α-dihydroxy-16α-methyl-pregnan-20-on wird in einem zweiten Reaktionsschritt mittels Chromsäure zum 5α-Brom-6β-fluor-17α-hydroxy-16α-methyl-pregnan-3,20-dion oxydiert. Dieser Reaktionsschritt kann unter den Bedingungen durchgeführt werden, die man konventionellerweise bei der Oxydation von Hydroxysteroiden zu Oxosteroiden mittels Chromsäure anwendet. (J. Fried et. al., Organic Reactions in Steroid Chemistry; van Nostrand Reinhold Comp., New York et. al., Vol. 1, 1972, 227ff). Besondes hohe Ausbeuten an Verfahrensprodukt erzielt man aber, wenn man die Oxidation mittels Chromsäure-Schwefelsäure in einem polaren Ether (Dioxan, Tetrahydrofuran etc.) Als Lösungsmittel anstelle von Aceton durchführt.

Aus dem so dargestellten 5α-Brom-6β-fluor-17α-hydroxy-16α-methyl-pregnan-3,20-dion wird in an sich bekannter Weise Bromwasserstoff abgespalten. Erfindungsgemäß wird diese Abspaltung mittels starken Säuren als Katalysatoren in einem polaren Lösungsmittel durchgeführt, da unter diesen Bedingungen gleichzeitig das 6α-Fluoratom isomerisiert wird. Geeignete Bedingungen sind beispielsweise die Umsetzung der Bromverbindung mittels Bromwasserstoff oder Chlorwasserstoff in polaren Lösungsmitteln wie Essigsäure.

Das so erhaltene 6α-Fluor-17α-hydroxy-16α-methyl-4-pregnen-3,20-dion wird in an sich bekannter Weise mit einer lebenden Kultur von Pilzen der Gattung Curvularia in der 11-Position hydroxyliert (US-Patent 2,658,023).

Zur Hydroxylierung geeignete Pilze der Gattung Curvularia sind beispielsweise Curvularia falcata QM-102.H, Curvularia genticulata IFO (6284) und Curvularia lunata NRRL 2380, NRRL 2434 oder ATCC 12017. Die Hydroxylierung kann unter den üblichen Bedingungen durchgeführt werden, wie sie beispielsweise im Europäischen Patent 000 3341 beschrieben sind.

Aus dem so hergestellten 6α-Fluor-11β,17α-dihydroxy-16α-methyl-4-pregnen-3,20-dion wird in dem nun folgenden Reaktionsschritt des erfindungsgemäßen Verfahren Wasser abgespalten. Diese Dehydratisierung kann unter den dem Fachmann wohl bekannten Bedingungen durchgeführt werden. Geeignete Bedingungen sind zum Beispiel die Dehydratisierung mittels Thionylchlorid in Pyridin, mittels Phosphoroxychlorid in Pyridin, oder mittels Methansulfonsäurechlorid und Schwefeldioxid in Dimethylformamid/Collidin. Als eine sehr brauchbare Methode hat sich auch die Umsetzung des 6α-Fluor-11β,17α-dihydroxy-16α-methyl-4-pregnen-3,20-dion mit N-Bromacylamiden (insbesondere N-Bromacetamid) in Pyridin und anschließende Behandlung der Reaktionsmischung mit Schwefeldioxid erwiesen. Die Bedingungen, unter denen diese Dehydratisierungsverfahren durchgeführt werden sind konventionell (J. Fried et. al. Organic Comp. New York et. al., Vol. 1, 1972, p 320ff).

An das so hergestellte 6α-Fluor-17α-hydroxy-16α-methyl-4,9,(11)-pregnadien-3,20-dion wird in üblicher Weise BrOH angelagert. Dies kann beispielsweise geschehen, indem man in Gegenwart von wasserhaltigen Lösungsmitteln (wie wasserhaltigem Aceton, Dioxan oder Tetrahydrofuran, N-Bromacylamide (N-Bromacetamid oder insbesondere N-Bromsuccinimid) auf diese Verbindungen einwirken läßt. Bekanntlich kann diese Reaktion unter Verwendung von Säuren (Salzsäure oder Perchlorsäure) als Katalysatoren durchgeführt werden.

Das so erhaltene 9α-Brom-6α-fluor-11β,17α-dihydroxy-16α-methyl-4-pregnen-3,20-dion wird in an sich bekannter Weise mittels Basen (Natriumhydroxid, Natriumacetat u.a.) in das 9β,11β-Epoxy-6α-fluor-17α-hydroxy-16α-methyl-4-pregnen-3,20-dion überführt. (J. Fried et. al. Organic Reactions in Steroid Chemistry; Van Nostrand Reinhold Comp. New York et. al.; Vol II, 1972, 15ff).

Der Epoxidring des 9β,11β-Epoxy-6α-fluor-17α-hydroxy-16α-methyl-4-pregnen-3,20-dion nun mittels Fluorwasserstoff geöffnet und man erhält das 6α,9α-Difluor-11β,17α-dihydroxy-16α-methyl-4-pregnen-3,20-dion. Die Bedingungen, unter denen diese Reaktionsstufe durchgeführt wird, sind ebenfalls konventionell (J. Fried et. al. Organic Reactions in Steroid Chemistry; Van Nostrand Reinhold Comp. New York et. al. Vol. 1, 1972, 425ff).

Gewünschtenfalls kann das erhaltene 6α,9α-Difluor-11β,17α-dihydroxy-16α-methyl-4-pregnen-3,20-dion nun mittels zur Steroid-$\Delta^1$-dehydrierung befähigter Mikroorganismen zum 6α,9α-Difluor-11β,17α-dihydroxy-16α-methyl-1,4-pregnadien-3,20-dion dehydriert werden. Geeignete Mikroorganismen zur Dehydrierung sind beispielsweise solche der Gattung Bacillus (wie zum Beispiel Bacillus lentus ATCC 13805, oder Bacillus sphaericus ATCC 7054 und ATCC 7055) oder der Gattung Arthrobacter (wie zum Beispiel Arthrobacter simplex ATCC 6945). Die Bedingungen unter denen dieser Reaktionsschritt durchgeführt werden kann sind konventionell und beispielsweise in den Europäischen Patenmtanmeldungen 0003341 oder 0054810 beschrieben.

Das erhaltene 6α,9α-Difluor-11β,17α-dihydroxy-16α-methyl-1,4-pregnadien-3,20-dion wird kann in an sich bekannter Weise in das entsprechende 21-Jod-Derivat überführt werden. Als besonders vorteilhaft hat

sich das StorkVerfahren der 21-Jod-Einführung erwiesen (J. Fried et. al.: Organic Reactions in Steroid Chemistry; Van Nostrand Reinhold Comp. New York,etc. Vol. II, 1972,207). die erhaltenen 21-Brom- oder 21-Jodverbindungen können gewünschtenfalls in die 21-Alkanoyloxy-6α,9α-difluor-11β,17α-dihydroxy-16α-methyl-1,4-pregnadien-3,20-dion der allgemeinen Formel Id überführt werden, indem man sie in Gegenwart von Basen mit den entsprechenden Säuren umsetzt (J. Fried et al.: Organic Reactions in Steroid Chem.; Van Nostrand Reinhold Comp. New York etc. Vol. II, 1972, 203-212 und 220-227). Als Säure wird vorzugsweise Essigsäure verwendet, die man beispielsweise in einem inerten Lösungsmittel wie Aceton in Gegenwart von Basen, wie Natriumacetat oder Triethylamin auf die 21-Halogenverbindung einwirken läßt.

Die Verseifung des 21-Acetats zum 6α,9α-Difluor-11β,17α,21-trihydroxy-16α-methyl-1,4-pregnadien-3,20-dion (Flumethason) ist vorbekannt.

Es wurde bereits erwähnt, daß es mit Hilfe des erfindungsgemäßen Verfahrens möglich ist, Flumethason auf wesentlich einfachere Weise und unter Erzielung höherer Ausbeuten herzustellen, als dies mittels der vorbekannten Synthesen möglich ist. Ein weiterer Vorzug des Erfindungsgemäßen Verfahrens besteht darin, daß bei den einzelnen Verfahrensschritten in der Regel die Verfahrensprodukte in so hoher Reinheit anfallen, (dies gilt insbesondere auch für die 11β-Hydroxylierung), daß aufwendige Reinigungskristallisationen vermieden werden können, welche bei derartig vielstufigen Synthesen meist erhebliche Ausbeutenverluste an Verfahrensendprodukt bewirken.

Das nachfolgende Ausführungsbeispiel dient zur näheren Erläuterung der Erfindung.

Beispiel

a) Zu einer Suspension von 180,7 g 3β,17α-Dihydroxy-16α-methyl-5-pregnen-20-on in 1445 ml Toluol gibt man in einem Polyethylengefäß unter Rühren bei 0° C 240,2 ml Harnstoff-Flußsäure-Reagenz (45:55 Gewichtsprozent) und anschließend 97,3 g N-Bromsuccinimid portionsweise so zu, daß die Temperatur 0 bis 3° C beträgt. Die Suspension wird 2 Stunden bei 0° C nachgerührt und dann zu einem Gemisch von 2 kg Eis und 1441 ml 25 %igem Ammoniak gegeben. Das Toluol wird mit Wasserdampf abdestilliert, die wässrige Suspension auf 20° C abgekühlt, das Reaktionsprodukt abgesaugt und mit ca. 700 ml Wasser gut nachgewaschen. Das wasserfeuchte Produkt wird erst mit 813 ml Essigester und dann noch zweimal mit je 500 ml Essigester ausgerührt, abgesaugt und bei 30° C im Umlufttrockenschrank getrocknet.

Ausbeute: 166 g 5α-Brom-6β-fluor-3β,17α-dihydroxy-16α-methyl-pregnan-20-on vom Schmelzpunkt 183 bis 185° C (Zers.).

b) Zu einer Suspension von 165 g 5α-Brom-6β-fluor-3β,17α-dihydroxy-16α-methyl-pregnan-20-on in 1655 ml Tetrahydrofuran und 58 ml Wasser gibt man 258 ml 8n Chromsäure-Lösung (hergestellt durch Lösen von 267,2 g Chrom(VI)-oxid in 230 ml konzentrierter Schwefelsäure und Verdünnen des Gemisches mit Wasser auf 1000 ml) so zu, daß die Temperatur 0 bis 2° C beträgt und rührt noch 2 Stunden bei dieser Temperatur nach. Dann gibt man 15 ml 2-Propanol hinzu, rührt weitere 10 Minuten und fällt das Reaktionsprodukt in 8,3 Liter Eiswasser, saugt ab und wäscht dreimal mit je 250 ml Wasser. Das wasserfeuchte Produkt wird in 414 ml Aceton 15 Minuten bei 20° C kräftig gerührt, abgesaugt und zweimal mit je 50 ml Aceton gewaschen. Eine im Vakuum vorsichtig getrocknete Probe des 5α-Brom-6β-fluor-17α-hydroxy-16α-methyl-pregnan-3,20-dions hat einen Schmelzpunkt von 105 bis 115° C (Zers.).

c) Das nutschenfeuchte 5α-Brom-6β-fluor-17α-hydroxy-16α-methyl-pregnan-3,20-dion wird in 1655 ml Essigsäure suspendiert, auf 30° C erwärmt und unter Rühren mit 5,36 ml Bromwasserstoff in Eisessig, 33 Gewichtsprozentig, versetzt. Nach ca. 30 Minuten tritt eine klare Lösung ein. Danach werden weitere 30 Minuten bei 30° C gerührt. Anschließend gibt man 35,1 g Natriumacetat hinzu und fällt in 6,62 Liter Eiswasser. Die Suspension wird 30 Minuten nachgerührt, das Reaktionsprodukt abgesaugt, zweimal mit einer Lösung von je 17,5 g Natriumacetat in 250 ml Wasser und einmal mit 250 ml Wasser gewaschen und bei 30° C im Umlufttrockenschrank getrocknet.

Ausbeute: 123 g 6α-Fluor-17α-hydroxy-16α-methyl-4-pregnen-3,20-dion vom Schmelzpunkt 168 bis 184° C (Zers.).

Nach dünnschichtchromatographischer Analyse auf Kieselgel-Platten im System Toluol/Isopropanol 15:3, drei Durchgänge, enthält das Reaktionsprodukt ca. 10 % 6β-Fluor-Isomeres.

d) Ein 2 Liter Erlenmeyerkolben mit 1 Liter steriler Nährlösung, enthaltend 1 % Cornsteep liquor und 1,25 % Sojapuder, eingestellt auf pH 6,2, wird mit einer Abschwemmung einer Trockenkultur von Curvularia lunata NRRL 2380 beimpft und bei 30° C 72 Stunden mit 175 Umdrehungen pro Minute geschüttelt.

Ein 50 Liter-Fermenter mit 30 Liter einer sterilen Nährlösung, enthaltend 1 % Cornsteep liquor, 1,25 % Sojapuder und 4 ml Silikon SH, eingestellt auf pH 6,5 wird mit 1 Liter der Curvularia lunata-Anzuchtskultur beimpft und diese Vorkultur bei 30° C unter Belüftung mit 2 $m^3$ Luft pro Stunde 150 Umdrehungen pro Minute 18 Stunden lang inkubiert.

Ein 50 Liter Fermenter wird mit 30 Liter steriler Nährlösung, enthaltend 1 % Cornsteep liquor, 1,25 % Sojapunder und 4 ml Silikon SH, eingestellt auf pH 6,5, beschickt, mit 3 Liter Curvularia lunata -Vorkultur beimpft und unter Belüften von 2 $m^3$ Luft pro

Stunde und Rühren mit 150 Umdrehungen pro Minute 5 Stunden lang bei 30° C inkubiert.

Dann setzt man der Kultur eine sterilfiltrierte Lösung von 12 g 6α-Fluor-17α-hydroxy-16α-methyl-4-pregnen-3,20-dion (72,1 %ig) in 600 ml Äthylenglykolmonomethylether zu und fermentiert weitere 35 Stunden . Die Belüftung beträgt nach der Substratzugabe weiter 2 $m^3$ Luft pro Stunde, das Rühren wird auf 220 Umdrehungen pro Minute erhöht.

g) Nach erfolgter Fermentation wird die Kulturbrühe dreimal mit je 20 Liter Methylisobutylketon extrahiert und der Methylisobutylketon-Extrakt im Rotationsverdampfer bei max. 50° C unter Vakuum eingeengt.

Nach Abdestillieren der gesamten Lösungsmittelmenge nimmt man den Rückstand in 100 ml Essigester unter Erwärmen auf dem Dampfbad auf und kühlt nach vollständiger Lösung des Rückstandes auf Raumtemperatur ab.

Das Kristallisat wird über einen Büchner-Trichter abgesaugt, mit Essigester nachgewaschen und bei 50° C im Vakuum-Trockenschrank getrocknet.

Man erhält 6,4 g 11β-17α-Dihydroxy-6α-fluor-16α-methyl-4-pregnen-3,20-dion (96 %ig). In der Mutterlauge wurden chromatographisch noch 1,5 g dieses Produkts bestimmt.

e) 100 g 6α-Fluor-11β,17α-dihydroxy-16α-methyl-4-pregnen-3,20-dion werden in 1050 ml trockenem Pyridin gelöst. Zu der Lösung gibt man bei 25° C 39,4 g N-Bromacetamid und rührt 15 Minuten nach. Man kühlt dann auf 10° C und leitet bei dieser Temperatur Schwefeldioxyd ein, bis die Probe auf angesäuertes Kaliumjodid-Stärke-Papier negativ verläuft. Anschließend werden 2100 ml Wasser bei 20° C zugetropft. Danach rührt man unter Eiswasserkühlung 15 Stunden nach. Der ausgefallene Niederschlag wird abfiltriert, mit Wasser neutral gewaschen und getrocknet. Es werden 95,5 g 6α-Fluor-17α-hydroxy-16α-methyl-4,9(11)-pregnadien-3,20-dion erhalten, das nach Umkristallisation aus Diisopropylether bei 219 bis 225° C schmilzt.

f) Zu einer Suspension von 50 g 6α-Fluor-17α-hydroxy-16α-methyl-4,9(11)-pregnadien-3,20-dion-Rohprodukt in 500 ml Aceton und 100 ml Wasser tropft man unter Kühlung eine Lösung von 31,7 g N-Bromsuccinimid. Man rührt weitere 2 Stunden nach. Nach Beendigung der Reaktion werden 1,1 Liter Wasser zum Reaktionsgemisch gegeben. Das ausgefallene 9α-Brom-6α-fluor-11β,17α,-dihydroxy-16α-methyl-4-pregnen-3,20-dion wird abgfiltriert, gewaschen und trocken gesaugt. Schmelzpunkt einer getrockneten Probe: 138 bis 143° C unter Zersetzung. Anschließend wird das wasserfeuchte 9α-Brom-6α-fluor-11β,17α-dihydroxy-16α-methyl-4-pregnen-3,20-dion in 400 ml Methanol suspendiert. Zur Suspension gibt man 50 g Natriumcarbonat und rührt bei erhöhter Temperatur nach. Man kühlt auf Raumtemperatur und fällt das 9,11β-Epoxy-6α-fluor-17α-hydroxy-16α-methyl-4-pregnen-3,20-dion durch Zugabe von Wasser aus. Es wird abfiltriert, mit Wasser neutral gewaschen und getrocknet. Man erhält 41,3 g vom Schmelzpunkt 210 bis 214° C.

50 g 9,11-Epoxy-6α-fluor-17α-hydroxy-16α-methyl-4-pregnen-3,20-dion werden in 175 ml Methylenchlorid suspendiert und unter Kühlung 175 ml Harnstoff-Flußsäure-Reagenz (45:55 Gewichtsprozent) zugegeben. Danach rührt man noch 2 Stunden bei Raumtemperatur. Nach Zugabe von 500 ml Eiswasser wird mit konz. Ammoniak neutalisiert und das Methylenchlorid mit Wasserdampf abdestilliert. Das Reaktionsprodukt wird abgesaugt, mit Wasser gewaschen, im Umlufttrockenschrank bei 50° C getrocknet und aus Methylenchlorid/Methanol umkristallisiert. Man erhält 38 g 6α,9α-Difluor-11β,17α-dihydroxy-16α-methyl-4-pregnen-3,20-dion, das bei 254 bis 258° C unter Zersetzung schmilzt.

h) Ein 2 Liter Erlenmeyerkolben mit 1 Liter steriler Nährlösung enthaltend 0,05 % Glucose, 1,0 % Hefeextrakt, 0,5 Cornsteep liquor, eingestellt auf pH 7,0, wird mit einer Abschwemmung von Bacillus lentus ATCC 13805 beimpft und bei 30° C 48 Stunden mit 180 Umdrehungen pro Minute geschüttelt.

Ein 50 Liter-Fermenter mit 30 Liter steriler Nährlösung, enthaltend 0,05 % Glucose, 1,0 % Hefeextrakt, 0,5 % Cornsteep liquor und 4 ml Silikon SH, eingestellt auf pH 7,0, wird mit 1 Liter der Bacillus lentus -Anzuchtskultur beimpft. Diese Vorkultur wird bei 30° C unter Belüftung von 2 $m^3$ Luft pro Stunde und Rühren mit 220 Umdrehungen pro Minute 24 Stunden lang inkubiert.

Ein 50 Liter-Fermenter wird mit 30 Liter steriler Nährlösung, enthaltend 0,05 % Glucose, 1,0 % Hefeextrakt, 0,5 % Cornsteep liquor und 4 ml Silikon SH, eingestellt auf pH 7,0, beschickt, mit 3 Liter Bacillus lentus-Vorkultur beimpft und unter Belüften von 2$m^3$ pro Stunde und Rühren mit 300 Umdrehungen pro Minute 6 Stunden bei 30° C inkubiert. 3 g 6α,9α-Difluor-11β,17α-dihydroxy-16α-methyl-4-pregnen-3,20-dion werden in 300 ml 2-Methoxy-ethanol zugesetzt und weitere 12 Stunden fermentiert.

Nach erfolgter Fermentation wird die mit 1 % Formalin sterilisierte Kulturbrühe dreimal mit je 20 Liter

Methylisobutylketon extrahiert und der Methylisobutylketon-Extrakt im Ratationsverdampfer bei max. 50° C unter Vakuum eingeengt.

Nach Abdestillieren der gesamten Lösungsmittelmenge nimmt man den Rückstand in 100 ml Essigester unter Erwärmen auf dem Dampfbad auf und kühlt nach vollständiger Lösung des Rückstandes auf Raumtemperatur ab.

Das Kristallisat wird über einen Büchner-Trichter abgesaugt, mit Essigester nachgewaschen und bei 50° C im Vakuum-Trockensschrank getrocknet.

Man erhält 2,6 g 6α,9α-Difluor-11β,17α-dihydroxy-16α-methyl-1,4-pregnadien-3,20-dion.

i) Zu einer Suspension von 10 g 6α,9α-Difluor-11β,17α-dihydroxy-16α-methyl-1,4-pregnadien-3,20-dion in 80 ml Methanol gibt man unter Stickstoff bei 25° C unter Rühren 10 ml einer gesättigten methanolischen Kalziumchloridlösung und 5,4 g Kalziumoxid. Dazu gibt man innerhalb von 1,5 Stunden eine Lösung von 21,6 g Jod und 3,2 g Kalziumchlorid in 62 ml Methanol und rührt drei Stunden bei 25° C nach. Dann fällt man in 300 ml Eiswasser, gibt 30 ml 10 %ige Essigsäure und anschließend 15 ml 10 %ige Natriumthiosulfatlösung zu und rührt 15 Minuten nach. Das Reaktionsprodukt wird abgesaugt und mit Wasser gut gewaschen.

Das wasserfeuchte 6α,9α-Difluor-11β,17α-dihydroxy-21-jod-16α-methyl-1,4-pregnadien-3,20-dion wird in 110 ml Aceton suspendiert. Man gibt 16,7 ml Essigsäure und 27,1 ml Triethylamin zur Reaktionsmischung und rührt 3,5 Stunden bei 50° C. Die Lösung wird im Vakuum auf ca. 75 ml eingeengt und in 500 ml Eiswasser gefällt. Man rührt die Suspension 1 Stunde nach, saugt das Reaktionsprodukt ab, wäscht mehrfach mit Wasser und trocknet bei 50° C im Umlufttrockenschrank. Man erhält so 11,2 g 21-Acetoxy-6α,9α-difluor-11β,17α-dihydroxy-16α-methyl-1,4-pregnadien-3,20-dion Schmelzpunkt 260 bis 264° C (aus Aceton/Diisopropylether).

## Patentansprüche

**1.** Verfahren zur Herstellung von 6α,9α-Difluor-11β,17α-dihydroxy-4-pregnen-3,20-dion und dessen Derivaten der allgemeinen Formel I

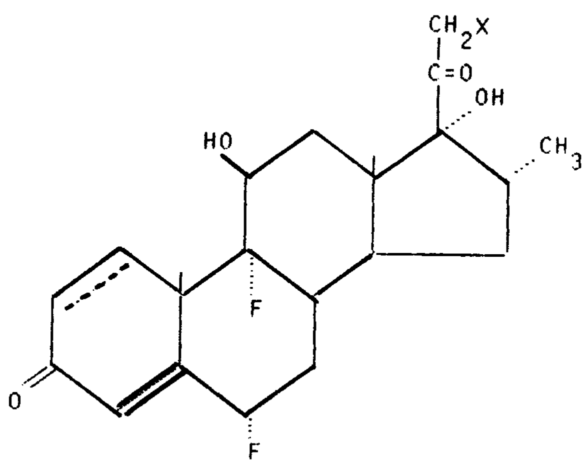

(I),

worin

..... eine Einfachbindung oder eine Doppelbindung symbolisiert und

X ein Wasserstoffatom, ein Bromatom, ein Jodatom oder eine Alkanoyloxygruppe mit maximal 8 Kohlenstoffatomen darstellt, dadurch gekennzeichnet, daß man das 3β,17α-Dihydroxy-16α-methyl-5-pregnen-20-on der Formel II

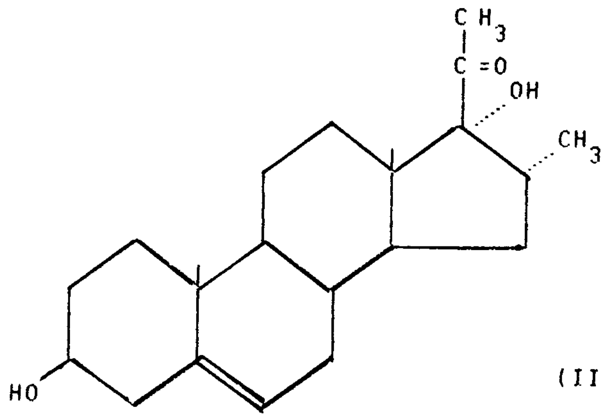

(II),

in Gegenwart von Harnstoff mit N-Brom-succinimid und Fluorwasserstoff umsetzt, das entstandene 5α-Brom-6β-fluor-3β,17α-dihydroxy-16α-methyl-pregnan-20-on der Formel III

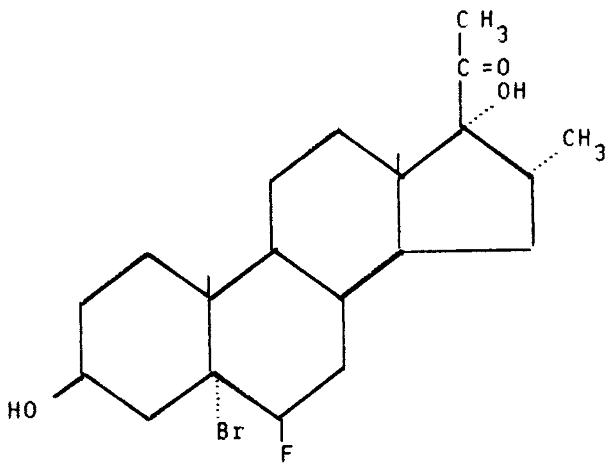

(III),

mittels Chromsäure zum 5α-Brom-6β-fluor-17α-hydroxy-16α-methyl-pregnan-3,20-dion der Formel IV

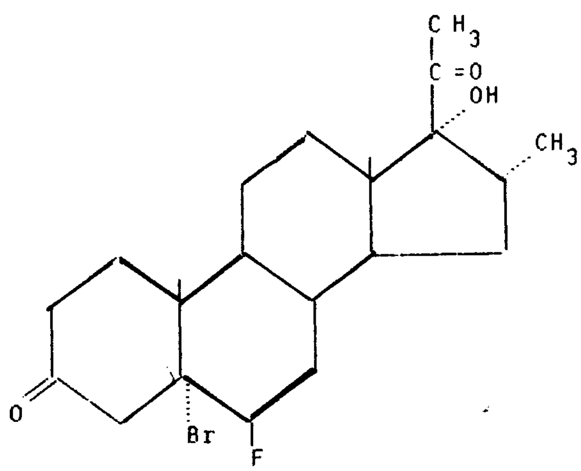

(IV),

oxidiert und aus diesem durch Behandeln mit einer starken Säure in einem polarem Lösungsmittel Bromwasserstoff abspaltet, wobei gleichzeitig eine Isomerisierung am C-6 stattfindet, das gebildete 6α Fluor-17α-hydroxy-16α-methyl-4-pregnen-3,20-dion der Formel V

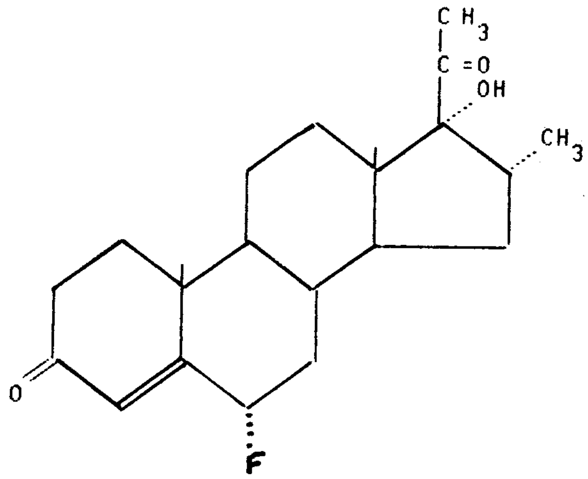

(V),

mit einer lebenden Kultur von Curvularia lunata in der 11-Position zum 6α-Fluor-11β,17α-dihydroxy-16α-methyl-4-pregnen-3,20-dion der Formel VI

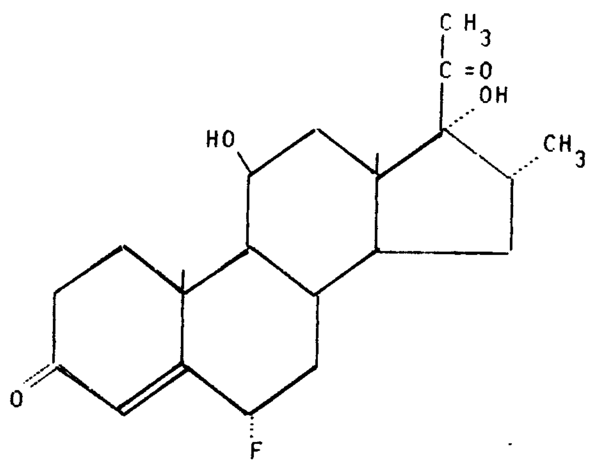

(VI),

hydroxyliert und aus diesem Wasser abspaltet, an das entstandene 6α-Fluor-17α-hydroxy-16α-methyl-4,(9),11-pregnadien-3,20-dion der Formel VII

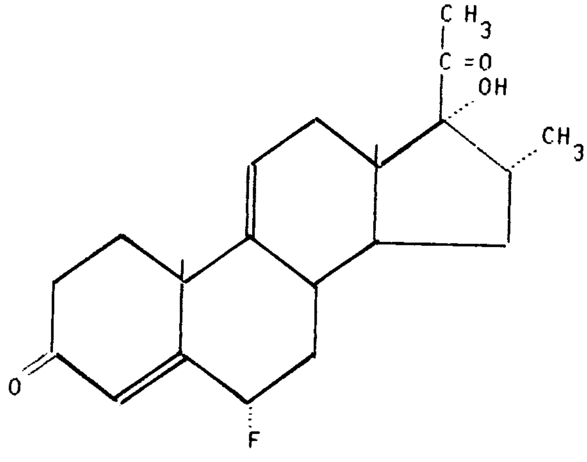

(VII),

BrOH anlagert, aus dem entstandenen 9α-Brom-6α-fluor-11β,17α-dihydroxy-16α-methyl-4-pregnen-3,20-dion der Formel VIII

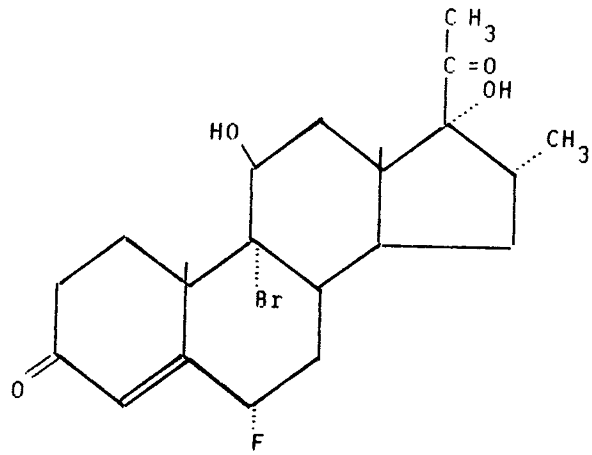

(VIII),

Bromwasserstoff abspaltet und den Epoxidring des gebildeten 9,11β-Epoxy-6α-fluor-17α-hydroxy-16α-methyl-4-pregnen-3,20-dions der Formel IX

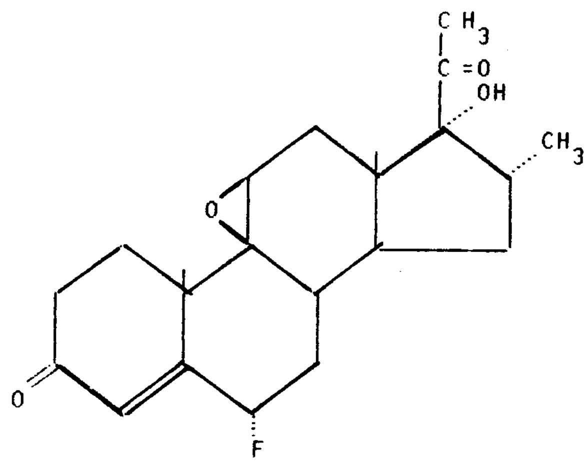

(IX),

mit Fluorwasserstoff öffnet, sowie gewünschtenfalls das erhaltene 6α,9α-Difluor-11β,17α-dihydroxy-16α-methyl-4-pregnen-3,20-dion der Formel Ia

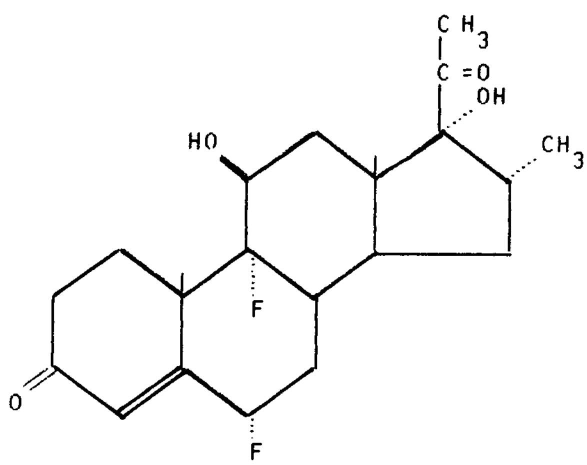

(Ia),

durch Fermentation mittels zur Steroid-$\Delta^1$-dehydrierung befähigten Mikroorganismen zum 6α,9α-Difluor-11β,17α-dihydroxy-16α-methyl-1,4-pregnadien-3,20-dion der Formel Ib

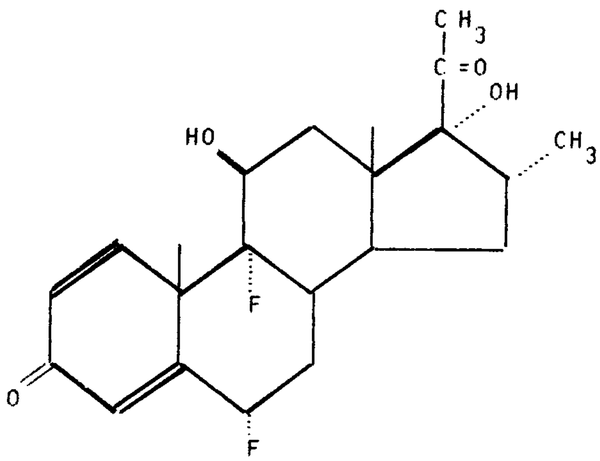

(Ib),

dehydriert, dieses mittels Brom oder Jod in das 6α,9α-Difluor-11β,17α-dihydroxy-21-halogen-16α-methyl-1,4-pregnadien-3,20-dion der Formel Ic

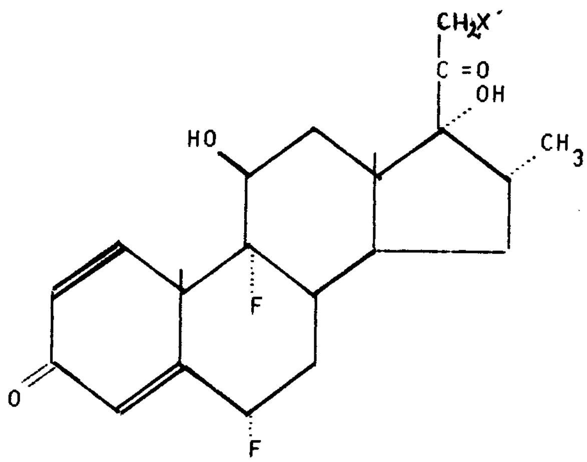

(Ic),

worin X' ein Bromatom oder Jodatom darstellt, überführt und aus diesem durch Austausch des Jods gegen eine Alkanoyloxygruppe das 21-Alkanoyloxy-6α,9α-difluor-11β,17α-dihydroxy-16α-methyl-1,4-pregnadien-3,20-dion der Formel Id

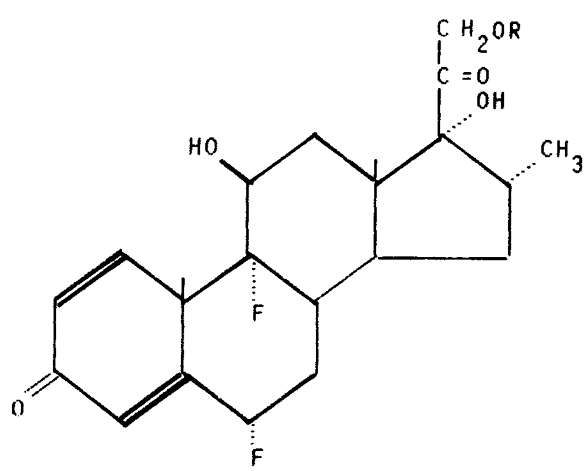

(Id),

worin R eine eine Alkanoylgruppe mit maximal 8 Kohlenstoffatomen bedeutet, darstellt.

**2.** 6α-Fluor-17α-hydroxy-16α-methyl-4,9(11)-pregnadien-3,20-dion.

**3.** 9α-Brom-6α-fluor-11β,17α-dihydroxy-16α-methyl-4-pregnen-3,20-dion.

**4.** 6α-Fluor-9β,11β-epoxy-17α-hydroxy-16α-methyl-4-pregnen-3,20-dion.

**Claims**

**1.** Process for the manufacture of 6α,9α-difluoro-11β,17α-dihydroxy-4-pregnene-3,20-dione and derivatives thereof of the general formula I

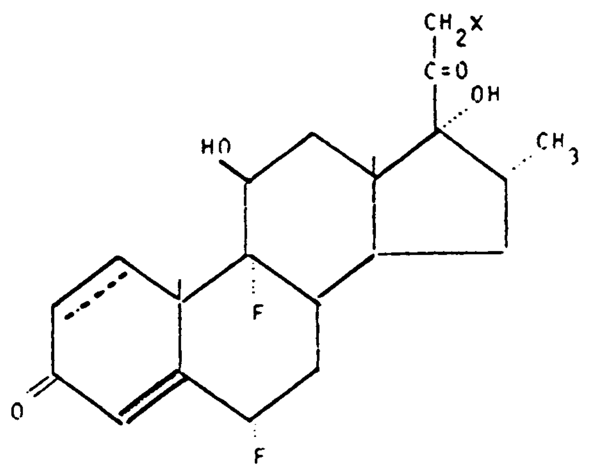

(I),

wherein

..... represents a single bond or a double bond and

X represents a hydrogen atom, a bromine atom, an iodine atom or an alkanoyloxy group having a maximum of 8 carbon atoms,

characterised in that 3β,17α-dihydroxy-16α-methyl-5-pregnen-20-one of the formula II

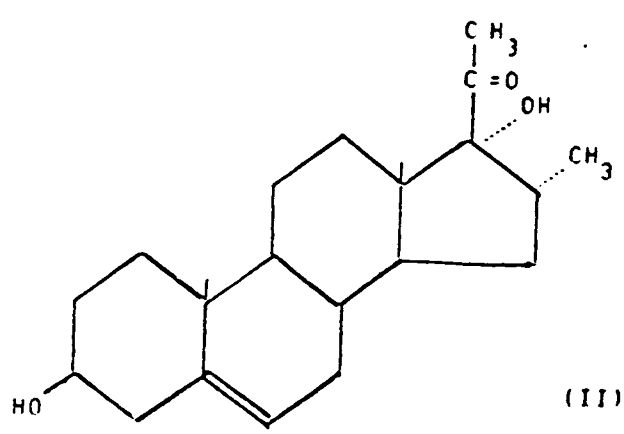

(II)

is reacted in the presence of urea with N-bromosuccinimide and hydrogen fluoride, the resulting 5α-bromo-6β-fluoro-3β,17α-dihydroxy-16α-methyl-pregnan-20-one of the formula III

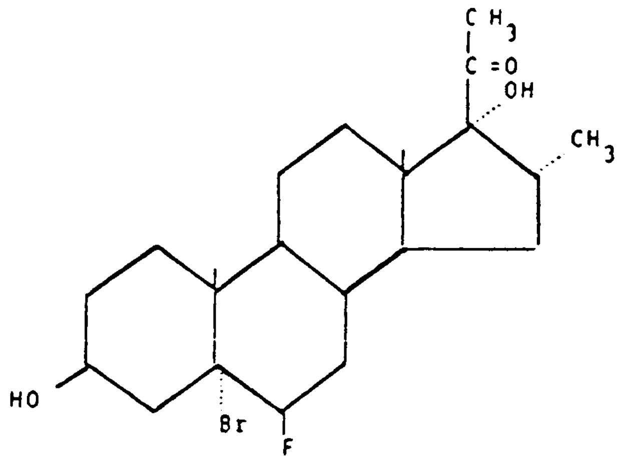

(III)

is oxidised by means of chromic acid to 5α-bromo-6β-fluoro-17α-hydroxy-16α-methyl-pregnane-3,20-dione of the formula IV

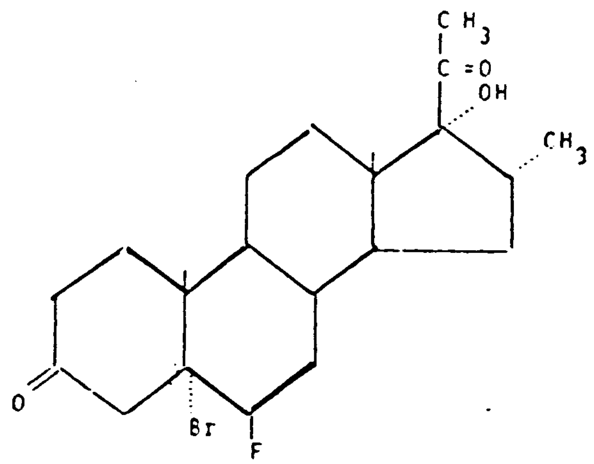

(IV).

and hydrogen bromide is split off from the latter by treatment with a strong acid in a polar solvent, with simultaneous isomerisation at the C-6 atom, the resulting 6α-fluoro-17α-hydroxy-16α-methyl-4-pregnene -3,20-dione of the formula V

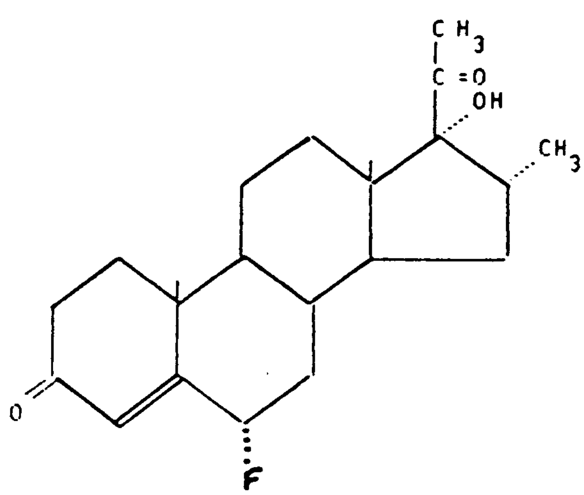

(V)

is hydroxylated in the 11-position with a living culture of Curvularia lunata to form 6α-fluoro-11β,17α-dihydroxy-16α-methyl-4-pregnene-3,20-dione of the formula VI

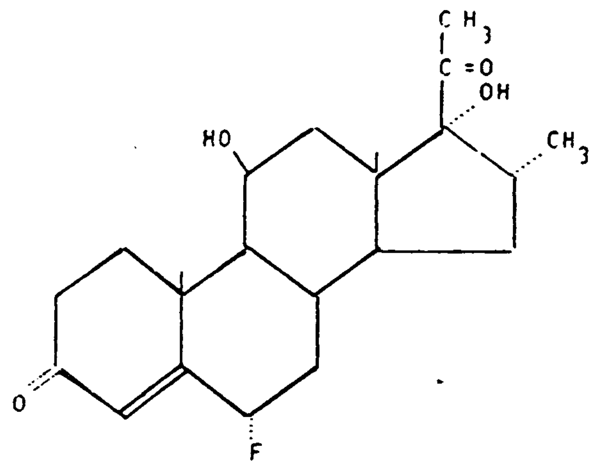

(VI),

and water is removed from the latter, BrOH is added to the resulting 6α-fluoro-17α-hydroxy-16α-methyl-4,(9),11-pregnadiene-3,20-dione of the formula VII

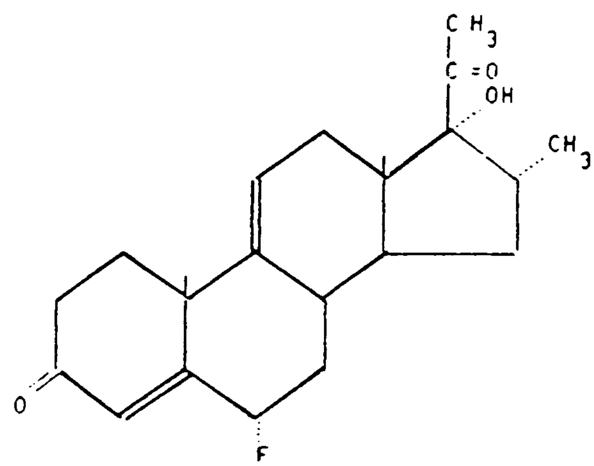

(VII),

hydrogen bromide is split off from the resulting 9α-bromo-6α-fluoro-11β,17α-dihydroxy-16α-methyl-4-pregnene-3,20-dione of the formula VIII

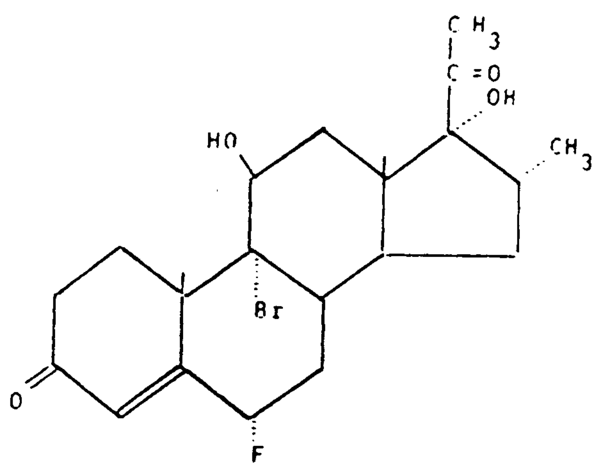

(VIII),

and the epoxy ring of the resulting 9,11β-epoxy-6α-fluoro-17α-hydroxy-16α-methyl-4-pregnene-3,20-dione of the formula IX

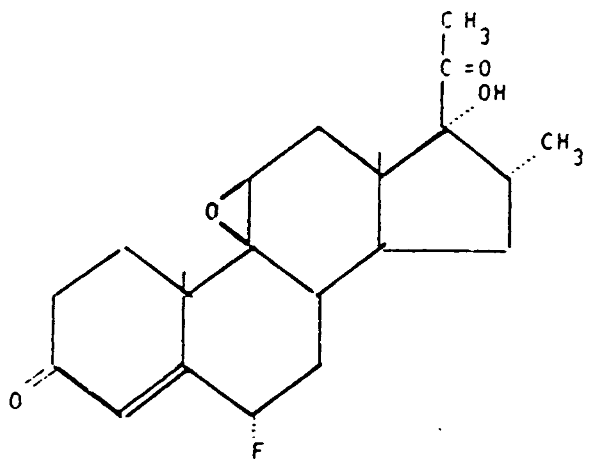

(IX)

is opened with hydrogen fluoride, and, if desired, the resulting 6α,9α-difluoro-11β,17α-dihydroxy-16α-methyl-4-pregnene-3,20-dione of the formula Ia

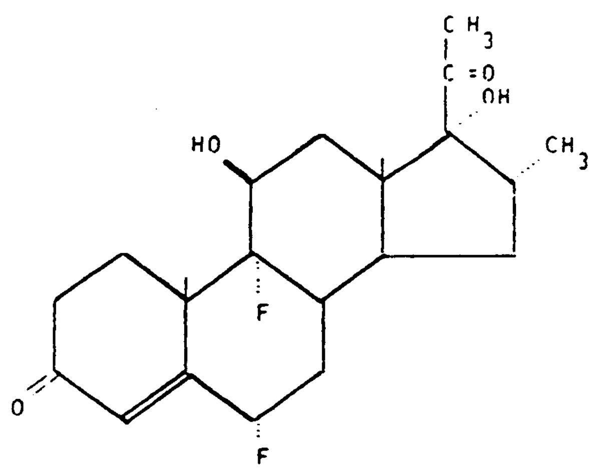

(Ia).

is dehydrogenated by fermentation by means of microorganisms capable of steroid $\Delta^1$-dehydrogenation to form 6α,9α-difluoro-11β,17α-dihydroxy-16α-methyl-1,4-pregnadiene-3,20-dione of the formula Ib

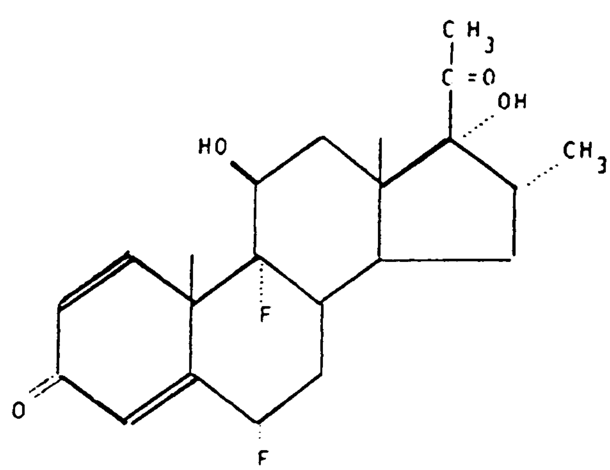

(Ib),

the latter is converted by means of bromine or iodine into 6α,9α-difluoro-11β,17α-dihydroxy-21-halo-16α-methyl-1,4-pregnadiene-3,20-dione of the formula Ic

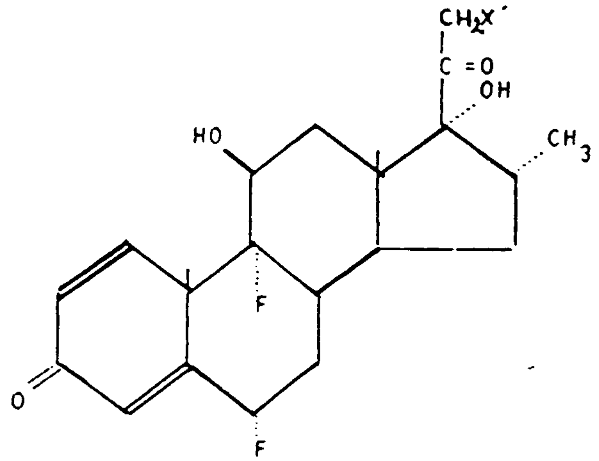

(Ic),

wherein X' represents a bromine atom or an iodine atom, and there is formed from the latter, by replacing the iodine by an alkanoyloxy group, 21-alkanoyloxy-6α,9α-difluoro-11β,17α-dihydroxy-16α-methyl-1,4-pregnadiene-3,20-dione of the formula Id

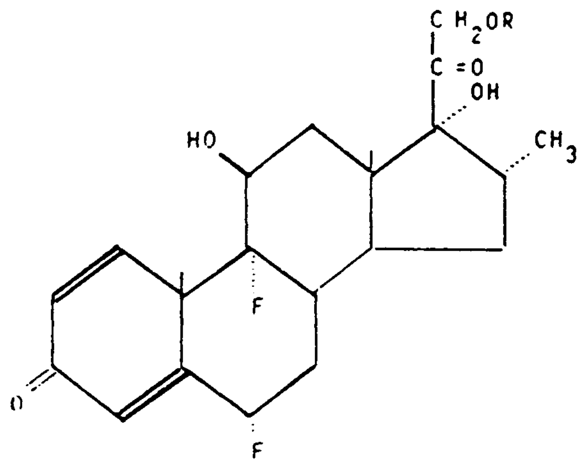

(Id),

wherein R represents an alkanoyl group having a maximum of 8 carbon atoms.

**2.** 6α-Fluoro-17α-hydroxy-16α-methyl-4,9(11)-pregnadiene-3,20-dione.

**3.** 9α-Bromo-6α-fluoro-11β,17α-dihydroxy-16α-methyl-4-pregnene-3,20-dione.

**4.** 6α-Fluoro-9β,11β-epoxy-17α-hydroxy-16α-methyl-4-pregnene-3,20-dione.

## Revendications

**1.** Procédé de préparation de la 6α,9α-difluoro-11β,17α-dihydroxy-4-prégnène-3,20-dione et de ses dérivés de formule générale I ci-dessous :

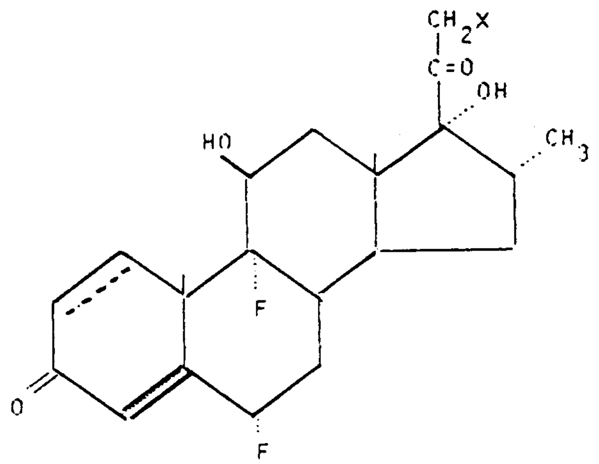

(I),

dans laquelle

..... symbolise une liaison simple ou une double liaison et

X désigne un atome d'hydrogène, de brome ou d'iode ou un groupe alcanoyloxy ayant au maximum 18 atomes de carbone, procédé caractérisé en ce que l'on fait réagir la 3β,17α-dihydroxy-16α-méthyl-5-prégnène-20-one de formule II :

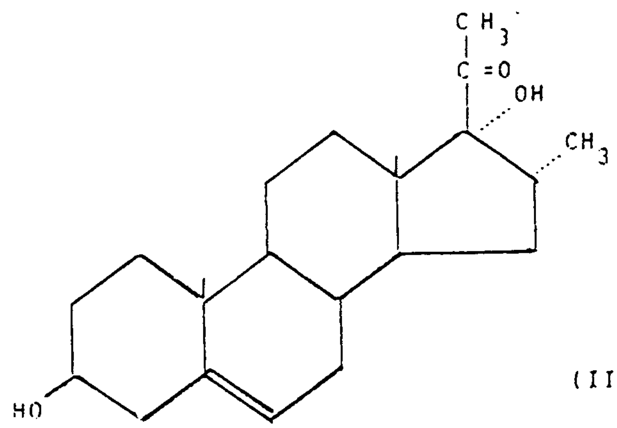

(II),

en présence d'urée avec le N-bromo-succinimide et du fluorure d'hydrogène, on oxyde ensuite la 5α-bromo-6β-fluoro-3β,17α-dihydroxy-16α-méthyl-prégnane-20-one ainsi formée, de formule III

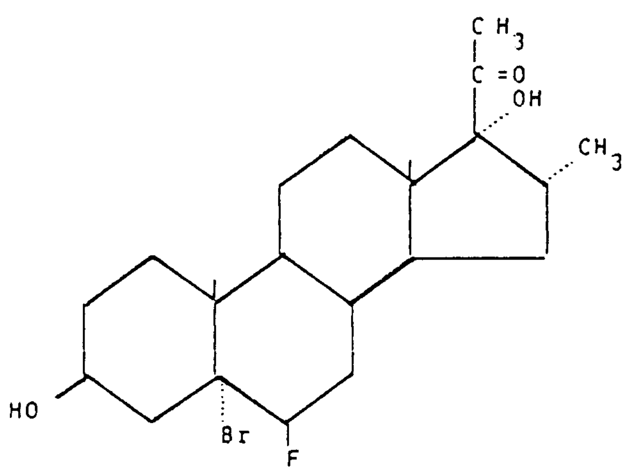

(III),

avec de l'acide chromique, en 5α-bromo-6β-fluoro-17α-hydroxy-16α-méthyl-prégnane-3,20-dione de formule IV

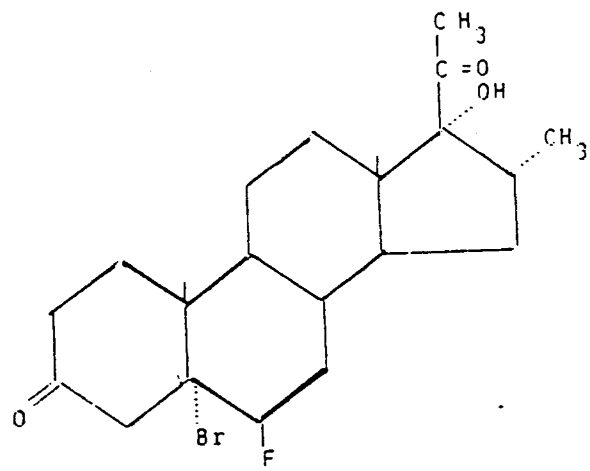

(IV),

dont on élimine une mole de bromure d'hydrogène en la traitant avec un acide fort dans un solvant polaire, ce qui produit simultanément une isomérisation à l'atome de carbone 6, on hydroxyle à la position 11 la 6α-fluoro-17α-hydroxy-16α-méthyl-4-prégnène,3,20-dione ainsi formée, de formule V

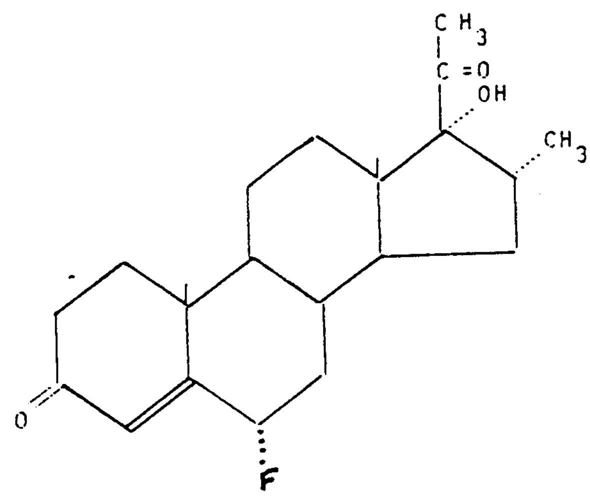

(V),

avec une culture vivante de Curvularia lunata, en 6α-fluoro-11β,17α-dihydroxy-16α-méthyl-4-prégnène-3,20-dione de formule VI

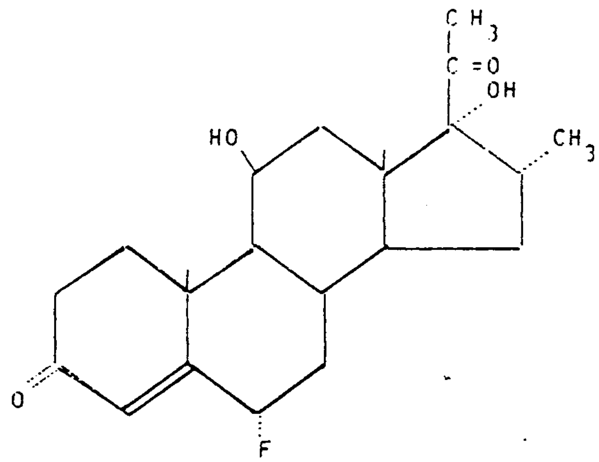

(VI),

dont on élimine une mole d'eau, on fixe une mole de BrOH sur la 6α-fluoro-17α-hydroxy-16α-méthyl-4,-(9),11-prégnadiène-3,20-dione de formule VII ainsi formée

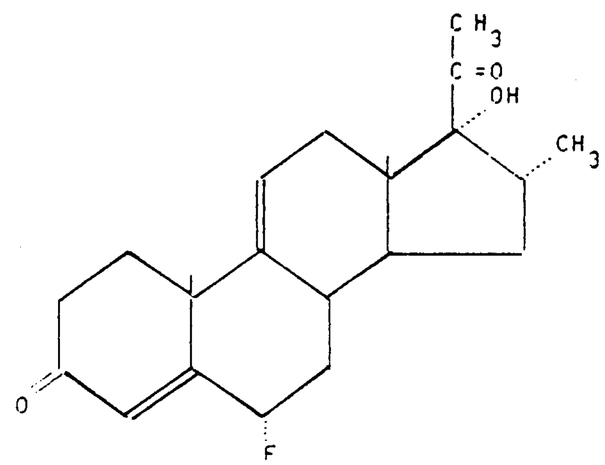

(VII),

on élimine ensuite une mole de bromure d'hydrogène de la 9α-bromo-6α-fluoro-11β,17α-dihydroxy-16α-méthyl-4-prégnène-3,20-dione formée, de formule VIII

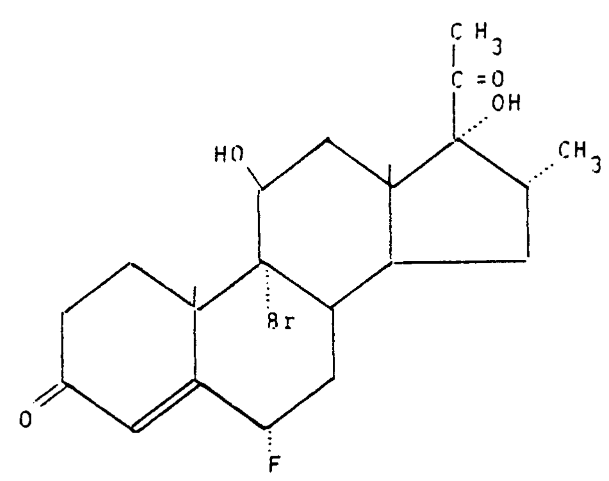

(VIII),

puis avec du fluorure d'hydrogène on ouvre le cycle époxydique de la 9,11β-époxy-6α-fluoro-17α-hydroxy-16α-méthyl-4-prégnène-3,20-dione de formule IX obtenue

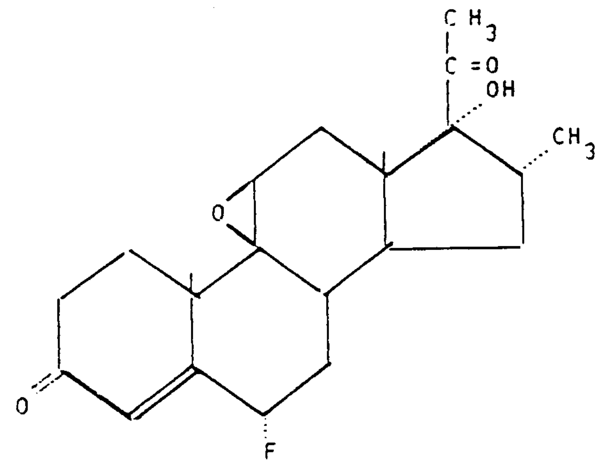

(IX),

et si on le souhaite on déshydrogène la 6α,9α-difluoro-11β,17α-dihydroxy-16α-méthyl-4-prégnène-3,20-dione ainsi obtenue de formule Ia

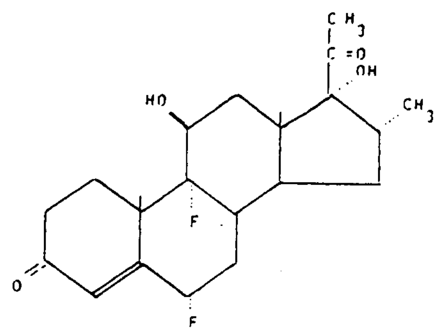

(Ia),

par fermentation avec des micro-organismes pouvant déshydrogèner un stéroïde-$\Delta^1$, pour former la 6α,9α-difluoro-11β,17α-dihydroxy-16α-méthyl-1,4-prégnadiène-3,20-dione de formule Ib

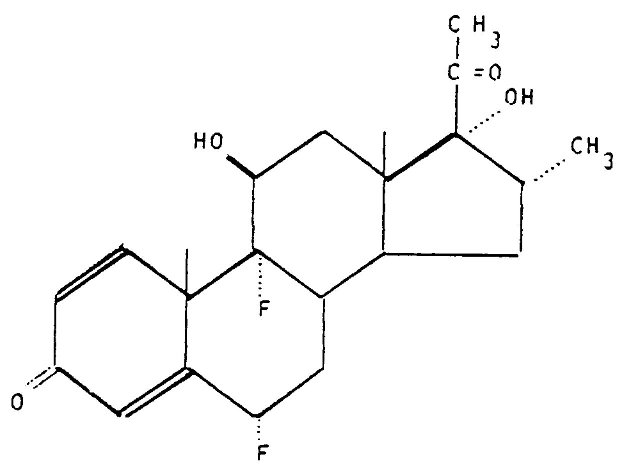

(Ib),

que l'on transforme avec du brome ou de l'iode en 6α,9α-difluoro-11β,17α-dihydroxy-21-halogéno-16α-méthyl-1,4-prégnadiène-3,20-dione de formule Ic

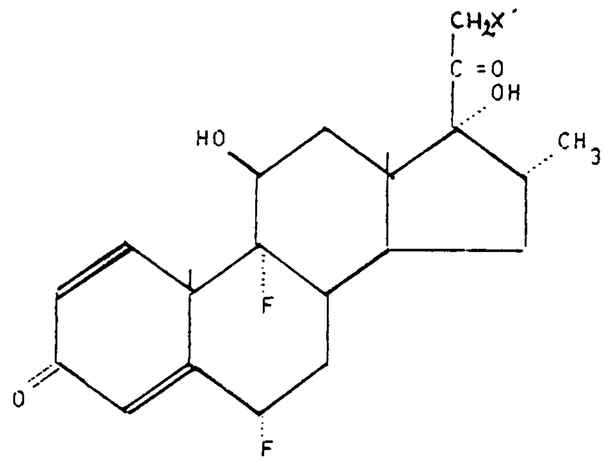

(Ic),

X' représentant un atome de brome ou d'iode, à partir de laquelle, par échange de l'iode ou du brome contre un groupe alcanoyloxy, on obtient la 21-alcanoyloxy-6α,9α-difluoro-11β,17α-dihydoxy-16α-méthyl-1,4-prégnadiène-3,20-dione de formule Id

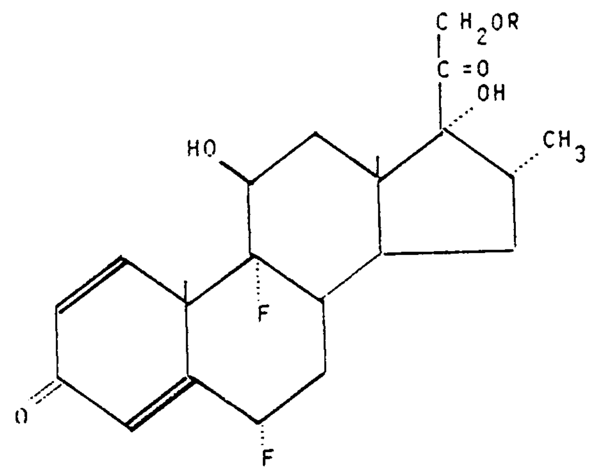

(Id),

dans laquelle R représente un groupe alcanoyle ayant au maximum 8 atomes de carbone.

**2.** La 6α-fluro-17α-hydroxy-16α-méthyl-4,9(11)-prégnaidène-3,20-dione

**3.** La 9α-bromo-6α-fluoro-11β,17α-dihydroxy-16α-méthyl-4-prégnène-3,20-dione.

**4.** La 6α-fluoro-9β,11β-époxy-17α-hydroxy-16α-méthyl-4-prégnène-3,20-dione.